# EUROPEAN PATENT APPLICATION

(11) **EP 1 647 602 A1**
(43) Date of publication of application: **19.04.2006**
(21) Application number: 05256313.7
(22) Date of filing: 11.10.2005
(51) Int. Cl.: C12Q 1/68

(54) **Array-based comparative genome hybridization assays**

(30) Priority: 12.10.2004 US 964505
(71) Applicant: Agilent Technologies, Inc., Palo Alto, CA 94306 (US)
(72) Inventor: Curry, Bo U., Redwood City California 94061 (US); Anderson, Paige L., Belmont California 94002 (US); Scheffer, Alicia F., Redwood City California 94061 (US); Ilsley, Diane D., San Jose California 95123 (US); Barrett Michael T., Mountain View California 94040 (US)
(74) Representative: Tollett, Ian

(57) **Abstract**

Methods and compositions for producing a labeled population of nucleic acids for use in an array-based comparative genome hybridization (CGH) assay are provided. In general, the methods involve cleaving a genomic source to produce a sample containing nucleic acid fragments, and end-labeling the nucleic acid fragments to provide a population of nucleic acids having a terminal label. Kits and systems for use in practising the subject methods are also provided.

## Description

This application relates to array-based comparative genome hybridization assays and, in particular, to methods and compositions for reducing label variation in array-based comparative genome hybridization assays.

Variations in the copy number of genomic sequences are associated with a variety of diseases and conditions. For example, gains and losses of genomic sequences up to and including whole chromosomes occur in many malignancies, e.g., colon cancer (Rajagopalan et al., Nature Cancer Review (2003) 3:695-701; Rabinovitch et al, Cancer Res. (1999) 59:5148-5153). As a result, tumor cells frequently have aneuploid genomes containing variable numbers of chromosomes and genetic content that deviates significantly from the normal diploid DNA content of non-neoplastic cells. Furthermore, genetic disorders frequently result from loss or gain of chromosomal regions. For example, in humans, trisomy of chromosome 21 results in Down's syndrome, trisomy of chromosome 13 results in Patau syndrome and abnormal numbers of sex chromosomes result in various developmental disorders, while abnormalities on the long arm of chromosome 18 are associated with 18q deletion syndrome.

Comparative genomic hybridization (CGH) is one approach that has been employed to evaluate variations in copy number of genomic regions in cells. In one implementation of CGH, genomic DNA is isolated from normal reference cells, as well as from test cells (e.g., tumor cells). The two nucleic acids are differentially labeled and then simultaneously hybridized *in situ* to metaphase chromosomes of a reference cell. Chromosomal regions in the test cells which are at increased or decreased copy number can be identified by detecting regions where the ratio of signal from the two distinguishably labeled nucleic acids is altered. For example, those regions that have been decreased in copy number in the test cells will show relatively lower signal from the test nucleic acid than the reference compared to other regions of the genome. Regions that have been increased in copy number in the test cells will show relatively higher signal from the test nucleic acid.

In a recent variation of the above traditional CGH approach, the immobilized chromosome element has been replaced with a collection of solid support surface-bound polynucleotides, e.g., an array of surface-bound BAC, cDNA or oligonucleotide probes for regions of a genome. Such approaches offer benefits over immobilized chromosome approaches, including a higher resolution, as defined by the ability of the assay to localize chromosomal alterations to specific areas of the genome.

However, despite the success of array-based CGH assays, the observed results obtained from such assays do not always accurately reflect the actual abundance of genomic regions in the sample. One difference between observed and expected results, termed herein as "signal bias" can, under certain circumstances, cause an array-based CGH assay to yield inaccurate results.

Accordingly, improved methods of reducing signal bias in array-based CGH assays are needed in order to more accurately and reliably evaluate copy number of a chromosomal region in a cell. This invention meets this, and other, needs.

### Relevant Literature

United States Patents of interest include: 6,465,182; 6,335,167; 6,251,601; 6,210,878; 6,197,501; 6,159,685; 5,965,362; 5,830,645; 5,665,549; 5,447,841 and 5,348,855. Also of interest are published United States Application 2002/0006622 and published PCT application WO 99/23256. Articles of interest include: Pollack et al. (Proc. Natl. Acad. Sci. (2002) 99: 12963-12968); Wilhelm et al. (Cancer Res. (2002) 62: 957-960); Pinkel et al. (Nat. Genet. (1998) 20: 207-211); Cai et al. (Nat. Biotech. (2002) 20: 393-396); Snijders et al. (Nat. Genet. (2001) 29:263-264); Hodgson et al. (Nat. Genet. (2001) 29:459-464); Trask (Nat. Rev. Genet. (2002) 3: 769-778); Rabinovitch et al. (Cancer Res. (1999) 59:5148-5153); and Lee et al. (Human Genet. (1997) 100:291:304).

According to a first aspect of the present invention, there is provided a method of producing a population of labeled nucleic acids for use in a comparative genome hybridization assay as claimed in claim 1.

According to a second aspect of the present invention, there is provided a comparative genome hybridization assay as claimed in claim 7.

According to a third aspect of the invention, there is provided a kit as claimed in claim 8.

According to a fourth aspect of the present invention, there is provided a system for performing a comparative genome hybridization assay as claimed in claim 9.

According to a fifth aspect of the present invention, there is provided a composition as claimed in claim 10.

Methods and compositions for producing a labeled population of nucleic acids for use in an array-based comparative genome hybridization (CGH) assay are provided. In general, the methods involve cleaving a genomic source to produce a sample containing nucleic acid fragments, and end-labeling the nucleic acid fragments to provide a population of nucleic acids having a terminal label. The terminal label is situated distal to the substrate surface when the labeled nucleic acids are hybridized to polynucleotides immobilized on that surface. The subject methods and compositions may be used to assess copy number of a genomic region, and, as such, may be employed in a variety of diagnostic and research applications. Kits and systems for use in practicing the subject methods are also provided.

Embodiments of the present invention are described below, by way of example only and with references to the accompanying drawings, in which:
Fig. 1 is a schematic representation of an exemplary end-labeled nucleic acid fragment complexed with a substrate surface-bound polynucleotide such that the terminal label of the end labeled nucleic acid fragment is distal to the substrate surface;
Fig. 2 is a schematic representation of an exemplary embodiment of the subject invention;
Fig. 3 is a schematic representation of an exemplary 3' end-labeled nucleic acid fragment complexed with a substrate surface-bound oligonucleotide such that the terminal label of the end labeled nucleic acid fragment is distal to the substrate surface; and
Fig. 4 is a schematic representation comparative genome hybridization assay according to certain embodiments of the subject invention.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other nucleic acids that are C-glycosides of a purine or pyrimidine base, polypeptides (proteins), polysaccharides (starches, or polysugars), and other chemical entities that contain repeating units of like chemical structure.

The term "nucleic acid" and "polynucleotide" are used interchangeably herein to describe a polymer of any length composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length. Oligonucleotides are usually synthetic and, in many embodiments, are under 50 nucleotides in length.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "surface-bound polynucleotide" refers to a polynucleotide that is immobilized on a surface of a solid substrate, where the substrate can have a variety of configurations, e.g., a sheet, bead, or other structure. In certain embodiments, the collections of oligonucleotide target elements employed herein are present on a surface of the same planar support, e.g., in the form of an array.

The phrase "labeled population of nucleic acids" refers to mixture of nucleic acids that are detectably labeled, e.g., fluorescently labeled, such that the presence of the nucleic acids can be detected by assessing the presence of the label. A labeled population of nucleic acids is "made from" a chromosome source, the chromosome source is usually employed as template for making the population of nucleic acids.

The term "array" encompasses the term "microarray" and refers to an ordered array presented for binding to nucleic acids and the like.

An "array," includes any one-dimensional, two-dimensional, substantially two-dimensional or three-dimensional arrangement of spatially addressable regions bearing nucleic acids, particularly oligonucleotides or synthetic mimetics thereof, and the like. Where the arrays are arrays of nucleic acids, the nucleic acids may be adsorbed, physisorbed, chemisorbed, or covalently attached to the arrays at any point or points along the nucleic acid chain.

Any given substrate may carry one, two, four or more arrays disposed on a front surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 cm² or even less than 10 cm², e.g., less than about 5cm², including less than about 1 cm², less than about 1 mm², e.g., 100 µm², or even smaller. For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 cm², or even less than 50 cm², 5 cm², 1 cm², 0.5 cm², or 0.1 cm². In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparentto reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either nucleic acid precursor units (such as monomers) in the case of *in situ* fabrication, or the previously obtained nucleic acid. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Inter-feature areas need not be present, particularly when the arrays are made by photolithographic methods as described in those patents.

An array is "addressable" when it has multiple regions of different moieties (e.g., different oligonucleotide sequences) such that a region (i.e., a "feature" or "spot" of the array) at a particular predetermined location (i.e., an "address") on the array will detect a particular sequence. Array features are typically, but need not be, separated by intervening spaces. In the case of an array in the context of the present application, the "population of labeled nucleic acids" will be referenced as a moiety in a mobile phase (typically fluid), to be detected by "surface-bound polynucleotides" which are bound to the substrate at the various regions. These phrases are synonymous with the terms "target" and "probe", or "probe" and "target", respectively, as they are used in other publications.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower'' are used in a relative sense only.

The term "stringent assay conditions" as used herein refers to conditions that are compatible to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. Stringent assay conditions are the summation or combination (totality) of both hybridization and wash conditions.

A "stringent hybridization" and "stringent hybridization wash conditions" in the context of nucleic acid hybridization (e.g., as in array, Southern or Northern hybridizations) are sequence dependent, and are different under different experimental conditions. Stringent hybridization conditions that can be used to identify nucleic acids within the scope of the invention can include, e.g., hybridization in a buffer comprising 50% formamide, 5xSSC, and 1% SDS at 42°C, or hybridization in a buffer comprising 5×SSC and 1% SDS at 65°C, both with a wash of 0.2xSSC and 0.1% SDS at 65°C. Exemplary stringent hybridization conditions can also include a hybridization in a buffer of 40% formamide, 1M NaCl, and 1% SDS at 37°C, and a wash in 1×SSC at 45°C. Alternatively, hybridization to filter-bound DNA in 0.5 M NaHPO₄, 7% sodium dodecyl sulfate (SDS), 1 mM EDTA at 65°C, and washing in 0. 1×SSC/0.1% SDS at 68°C can be employed. Yet additional stringent hybridization conditions include hybridization at 60°C or higher and 3 x SSC (450 mM sodium chloride/45 mM sodium citrate) or incubation at 42°C in a solution containing 30% formamide, 1M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions may affect the degree to which nucleic acids are specifically hybridized to complementary probes. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2xSSC at a temperature of at least about 50°C or about 55°C to about 60°C for about I to about 20 minutes; or, multiple washes with a solution with a salt concentration of about 0.1 xSSC containing 0.1% SDS at 20 to 50°C for 1 to 15 minutes; or, equivalent conditions. Stringent conditions for washing can also be, e.g., 0.2×SSC/0.1% SDS at 42°C. In instances wherein the nucleic acid molecules are deoxyoligonucleotides (i.e., oligonucleotides), stringent conditions can include washing in 6xSSC/0.05% sodium pyrophosphate at 37°C (for 14-base oligos), 48°C (for 17-base oligos), 55°C (for 20-base oligos), and 60°C (for 23-base oligos). See Sambrook, Ausubel, or Tijssen (cited below) for detailed descriptions of equivalent hybridization and wash conditions and for reagents and buffers, e.g., SSC buffers and equivalent reagents and conditions.

A specific example of stringent assay conditions is rotating hybridization at 65°C in a salt based hybridization buffer with a total monovalent cation concentration of 1.5M (e.g., as described in U.S. Patent Application No. 09/655,482 filed on September 5, 2000, the disclosure of which is herein incorporated by reference) followed by washes of 0.5xSSC and 0.1×SSC at room temperature and 37°C.

Stringent hybridization conditions may also include a "prehybridization" of aqueous phase nucleic acids with complexity-reducing nucleic acids to suppress repetitive sequences. For example, certain stringent hybridization conditions include, prior to any hybridization to surface-bound polynucleotides, hybridization with Cot-1 DNA, or the like.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "mixture", as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not especially distinct. In other words, a mixture is not addressable. To be specific, an array of surface bound polynucleotides, as is commonly known in the art and described below, is not a mixture of capture agents because the species of surface bound polynucleotides are spatially distinct and the array is addressable.

"Isolated" or "purified" generally refers to isolation of a substance (compound, polynucleotide, protein, polypeptide, polypeptide, chromosome, etc.) such that the substance comprises the majority percent of the sample in which it resides. Typically in a sample a substantially purified component comprises 50%, preferably 80%-85%, more preferably 90-95% of the sample. Techniques for purifying polynucleotides and polypeptides of interest are well known in the art and include, for example, ion-exchange chromatography, affinity chromatography, flow sorting, and sedimentation according to density.

The term "assessing" and "evaluating" are used interchangeably to refer to any form of measurement, and includes determining if an element is present or not. The terms "determining," "measuring," and "assessing," and "assaying" are used interchangeably and include both quantitative and qualitative determinations. Assessing may be relative or absolute. "Assessing the presence of" includes determining the amount of something present, as well as determining whether it is present or absent.

The term "using" has its conventional meaning, and, as such, means employing, e.g. putting into service, a method or composition to attain an end. For example, if a program is used to create a file, a program is executed to make a file, the file usually being the output of the program. In another example, if a computer file is used, it is usually accessed, read, and the information stored in the file employed to attain an end. Similarly if a unique identifier, e.g., a barcode is used, the unique identifier is usually read to identify, for example, an object or file associated with the unique identifier.

If a surface-bound polynucleotide "corresponds to" a chromosomal region, the polynucleotide usually contains a sequence of nucleic acids that is unique to that chromosomal region. Accordingly, a surface-bound polynucleotide that corresponds to a particular chromosomal region usually specifically hybridizes to a labeled nucleic acid made from that chromosomal region, relative to labeled nucleic acids made from other chromosomal regions.

Methods and compositions for producing a labeled population of nucleic acids for use in an array-based comparative genome hybridization (CGH) assay are provided. In general, the methods involve cleaving a genomic source to produce a sample containing nucleic acid fragments, and end-labeling the nucleic acid fragments to provide a population of nucleic acids having a terminal label. The terminal label is situated distal to the substrate surface when the labeled nucleic acids are hybridized to polynucleotides immobilized on that surface. The subject methods and compositions may be used to assess copy number of a genomic region, and, as such, may be employed in a variety of diagnostic and research applications. Kits and systems for use in practicing the subject methods are also provided.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Although any methods, devices and materials similar or equivalent to those described herein can be used in the practice or testing of the invention, the preferred methods, devices and materials are now described.

All publications mentioned herein are incorporated herein by reference for the purpose of describing and disclosing the invention components that are described in the publications that might be used in connection with the presently described invention.

As summarized above, the present invention provides new methods for producing a labeled population of nucleic acids for use in comparative genome hybridization (CGH) assays. In further describing the present invention, the subject labeling methods will be described first, followed by a detailed description of the use of a subject labeled population of nucleic acids in an exemplary CGH assay. Finally, representative kits and systems for use in practicing the subject methods will be discussed.

### LABELING METHODS

As discussed briefly above, the subject labeling methods involve cleaving a genomic source to produce a sample containing nucleic acid fragments, and end-labeling the nucleic acid fragments to provide a population of nucleic acids having a terminal label. The terminal label is situated distal to the substrate surface when the labeled nucleic acids are hybridized to polynucleotides immobilized on that surface. In general terms, a single terminus of the nucleic acid fragments (i.e., either the 5' end or the 3' end of the nucleic acid fragments but not both the 5' and 3' ends) is labeled. Which terminus of the nucleic acid fragments is labeled (i.e., whether it is the 3' or the 5' end that is labeled) is determined by the orientation of the surface-bound polynucleotides present in the array to be used: the end that is labeled is the same as the end that anchors (i.e., links) the surface-bound polynucleotides to the array, covalently or non-covalently. In other words, if the 3' end of a surface-immobilized polynucleotide anchors that polynucleotide to a CGH array, then the population of nucleic acids to be contacted with that array should contain 3'-end labeled nucleic acids. Conversely, if the 5' end of a surface-immobilized polynucleotide anchors that polynucleotide to an array, then the population of nucleic acids to be contacted with that array should contain 5'-end labeled nucleic acids.

Employment of the subject methods in a CGH assay significantly reduces signal bias, where "signal bias" is the difference between an observed evaluation of the abundance of a particular genomic region in a sample and the actual abundance of that genomic region in the sample. Without wishing to be bound to any particular theory about how the subject invention provides improved results, the subject methods are thought to reduce signal bias by reducing a phenomenon termed "quenching", where "quenching" occurs when energy from an photon absorbed by a label is transferred to a nearby energy receptor molecule rather than being re-radiated as an optically detectable signal (e.g., a fluorescent signal). Accordingly, when quenching occurs, a label typically emits less signal than it would if quenching does not occur, potentially leading to an underestimation of the amount of label present. Susceptibility to quenching varies between different labels, and different receptor molecules likely have different potentials for receiving energy from a label. Accordingly, since multiple labels may be employed in CGH assays, as well as polynucleotide arrays having several different types of energy receptor molecules (e.g., different substrate surface-immobilized nucleic acids and substrate coatings that may vary in amount and composition), quenching is thought to be a major source of signal bias in CGH assays. Accordingly, any method that reduces the amount of quenching in a CGH assay represents a significant contribution to CGH arts.

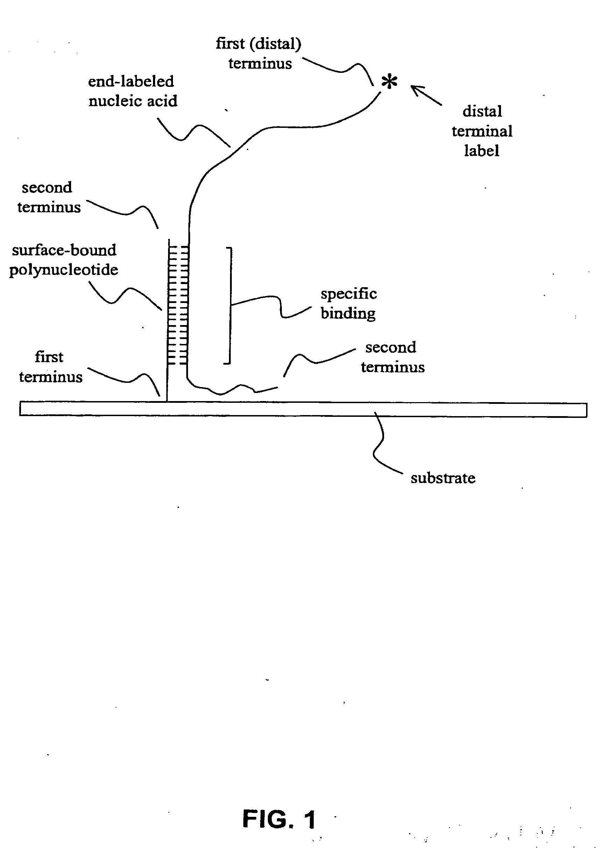

Fig. 1 shows an example of an end-labeled nucleic acid labeled according to the methods summarized above and described in greater detail below, specifically bound (i.e., hybridized) to a substrate surface-bound polynucleotide. In general terms, the surface-bound polynucleotide has a first terminus anchored to the surface of the substrate, and a second terminus that is distal to the substrate surface. The end labeled nucleic acid, when specifically bound to the surface-bound polynucleotide, is labeled at the same terminus as that anchoring the surface-bound polynucleotide. The labeled end of the end-labeled nucleic acid is therefore as far as possible from the surface when a hybrid is formed. In other words, if a surface-bound polynucleotide is bound to a substrate by its first terminus, then the end-labeled nucleic acid, when it is bound to the surface-bound polynucleotide, is also labeled at its first terminus (i.e., the same terminus that anchors the surface-bound polynucleotide to the surface of the substrate; the 3' or 5' terminus, whichever anchors the surface-bound polynucleotide to the surface of the substrate). The end-labeled nucleic acid contains a distal terminal label at the "distal terminus", where the term "distal terminus" is used to indicate the terminus of a nucleic acid that is the same as the terminus that anchor the surface-bound polynucleotide. In other words, if the surface-bound polynucleotide is anchored by its 5' end, then the distal terminus of the end-labeled nucleic acid is the 5' end of that nucleic acid, and if the surface-bound polynucleotide is anchored by its 3' end, then the distal terminus of the end-labeled nucleic acid is the 3' end of that nucleic acid. The term "distal terminus" may be used interchangeably with the term "distal end" in some descriptions of the invention.

Again without wishing to be bound to any particular theory about how the subject invention provides improved results, the subject methods are thought to reduce quenching because they provide for labeled nucleic acid/surface-bound polynucleotide complexes in which the labels of the bound nucleic acids are generally spatially distanced from energy receptor molecules (i.e., molecules that accept energy from labels during quenching) such that energy transfer occurs at a significantly reduced rate, as compared to equivalent complexes containing nucleic acids labeled by other means. In other words, the subject labeling methods are thought to produce labeled nucleic acids that, when bound to surface-bound polynucleotides, exhibit less quenching than nucleic acids labeled by other methods (e.g., methods that produce nucleic acids labeled at internal sites or at the opposite terminus to the distal terminus). Without any intention to be bound by any particular theory, the instant methods are thought to effectively reduce quenching by providing labeled nucleic acid/surface bound polynucleotide complexes in which the label, on average, is further away from energy receptor molecules than equivalent complexes containing nucleic acids labeled by other means. It is although thought that thermodynamic considerations, in particular the reduction of thermodynamic instabilities arising from labels attached to bases forming part of a double-stranded hybrid, or from labels in proximity to the array surface, may also play a role in the improved results ascribed to this invention. Further, the invention may improve results by providing populations of labeled nucleic acids that are more evenly labeled than those labeled by other methods. In particular, the number of labels associated with each species of labeled nucleic acid may be controlled to provide a population of labeled nucleic acid in which each labeled nucleic acid is associated with the same number of labels, e.g., one or two labels, for example.

In certain embodiments, therefore, the subject methods may be used to narrow the distribution of signals obtained from a CGH assay comparing two identical genomes by greater than 20%, greater than 30%, greater than 40%, greater than 50%, usually up to about 60% or more, using standard statistical test for measuring variance.

In describing the subject CGH labeling in more detail, suitable genomic sources will be described first, followed by a detailed description of how those genomic sources may be cleaved to provide a sample containing nucleic acid fragments, and labeled.

### Genomic sources

The subject methods involve labeling a genomic source using a specific end-labeling method described in greater detail below. In many embodiments a "genomic source" is a composition representing the nuclear genome of a cell, or a nucleic acid derivative thereof. For example, a genomic source may contain the entire complement of chromosomes of a cell (i.e., the chromosomes that make up the nuclear genome of a cell), fragmented versions thereof, amplified copies thereof, (-redundant). In particular embodiments, therefore, a genomic source contains amplified regions of a cellular genome, e.g., regions that hybridize to particular surface-bound polynucleotides. Many genomic sources are generally well known in the art since they have been described in other methods related to those described herein.

In certain embodiments, a genomic source may be of reduced complexity (usually at least at 20-fold less e.g., 25-fold less, at least about 50-fold less, at least about 75-fold less, at least about 90-fold less, or at least about 95-fold less complex in terms of total numbers of sequences present in the chromosome composition, as compared to the entire chromosome complement of a cell, up to and including a single gene regions ((e.g., introns and exons) being represented in the composition), as compared to the entire complement of chromosomes of a cell. In other words, in certain embodiments certain genomic sequences (e.g., repetitive and/or structural sequences) may be removed from a genome by affinity or other means, prior to initiation of the subject labeling methods. In other embodiments, particular genomic regions of interest may be amplified by known methods prior to initiation of the subject methods. In other embodiments, the genomic source does not have reduced (i.e., has nonreduced) complexity.

In general, the cells used in the subject methods may be any cell of interest, including any cell that contains or is suspected of containing a genomic region having abnormal copy number. Accordingly, cells from yeast, plants and animals, such as fish, birds, reptiles, amphibians and mammals may be used in the subject methods. In certain embodiments, mammalian cells, i.e., cells from mice, rabbits, primates, or humans, or cultured derivatives thereof, may be used.

In certain embodiments, a genomic source may represent a complex genome of at least about 1×10⁸ base pairs, including at least about 1×10⁹ base pairs, e.g., about 3×10⁹ base pairs. The average size of the constituent molecules that make up a genomic source may vary greatly. In certain embodiments, the constituent molecules have an average size of at least about 1 Mb, where a representative range of sizes is from about 50 Mb to about 250 Mb or more. In other embodiments, the sizes may not exceed about 1 MB, such that they may be about 1 Mb or smaller, e.g., less than about 500 Kb, etc. Since the subject methods involve a cleavage step that will reduce the average size of the constituent molecules in the genomic source, the precise size of the constituent molecules in the genomic source is not critical to the invention. Accordingly, such molecules may be in the region of 1 kb -50 Mb, and may be double or single stranded, depending on which labeling method is employed.

In certain embodiments, two genomic sources are labeled using the subject methods, and, as will be described in greater detail below, those labeled genomic sources may be compared using an array-based comparative genome hybridization assay. Accordingly genomic sources from a test and reference cell pair may be employed in the subject methods. The test and reference cells of a test and reference cell pair may be any two cells. However, in many embodiments, one cell of the pair has or is suspected of having a different phenotype or genotype compared to the other cell. In a particular embodiment, test and reference cell pairs include cancerous cells, e.g., cells that exhibit increased genomic instability, and non-cancerous cells, respectively or cells obtained from a sample of tissue from a test subject, e.g., a subject suspected of having a chromosome region copy number abnormality, and cells obtained from a normal, reference subject, respectively. A genomic source may be prepared from a subject, for example a plant or an animal, which subject is suspected of being homozygous or heterozygous for a deletion or amplification of a genomic region.

The genomic source may be prepared using any convenient protocol. In many embodiments, the genomic source is prepared by first obtaining a starting composition of genomic DNA, e.g., a nuclear fraction of a cell lysate, where any convenient means for obtaining such a fraction may be employed and numerous protocols for doing so are well known in the art (see, e.g., the methods for extracting genomic DNA described in Ausubel et al, Short Protocols in Molecular Biology, 3rd ed., Wiley & Sons, 1995; and Sambrook et al, Molecular Cloning: A Laboratory Manual, Third Edition, 2001 Cold Spring Harbor, N.Y.).

The genomic source is, in many embodiments of interest, genomic DNA representing the entire genome from a particular organism, tissue or cell type. However, in certain embodiments the genomic source may comprise a portion of the genome, e.g., one or more specific chromosomes or regions thereof, such as PCR amplified regions produced with a pairs of specific primers.

### Cleavage

In many embodiments of the subject labeling methods, a subject genomic source is cleaved, i.e., fragmented, to produce a sample containing nucleic acid fragments that serve as substrates for end-labeling. In general, at least 50% of the nucleic acid fragments in a cleaved sample may have a size range of about 100 bp to about 10 kb, e.g., 500 bp to 5 kb, as desired, although nucleic acid fragments having sizes outside of these ranges are readily employed in the subject methods.

Cleavage may be achieved using any convenient protocol, including but not limited to: mechanical protocols, e.g., sonication and shearing, etc., chemical protocols, e.g., cleavage by tris(3-hydroxy-1,2,3-benzotriazine-4(3*H*)one]iron(III) and other chemical agents, and enzymatic protocols, e.g., digestion by a restriction enzyme or the like. These methods are well known in the art.

In particular embodiments, one or more restriction enzymes (i.e., restriction endonucleases) may be employed in the subject cleavage methods. Suitable restriction enzymes may be chosen to be compatible with the labeling methods described in greater detail below. For example, restriction enzymes that cleave double stranded DNA asymmetrically to leave two, three or four single stranded bases ending in a 5' phosphate group (i.e., "sticky cutters") may be used if a 5' overhang is desirable, restriction enzymes that cleave double stranded DNA asymmetrically to leave two, three or four single stranded bases ending in a 3' hydroxyl group may be used if a 3' overhang is desirable, or restriction enzymes that cleave double stranded DNA symmetrically to leave no single stranded bases may be used if a flush or "blunt" end is desirable. Another criterion for the choice of restriction enzymes for use in the subject methods is the frequency at which they cleave the nucleic acids of a genomic source. As is well known in the art, restriction enzymes typically recognize a wide variety of four to six base-pair motifs (and larger), and may or may not be sensitive to the methylation status of a particular recognition motif.

Although virtually any restriction endonuclease may be used in the subject cleavage methods, it is most desirable to generate and label small nucleic acid fragments. Accordingly, restriction enzymes that recognize and cleave at a 4 base pair motif to produce a 5' overhang (e.g., Tsp509I, FatI, DpnII, Mbol, Sau3AI, MspI, HpaII, Taq I or MseI), a 3' overhang (e.g., Hhal, Tail or NlaIII), or a blunt end (e.g., AluI, BstUI, DpnI, HaeIII or RsaI) or any mixture thereof are of particular interest for use in the subject methods.

Restriction enzymes, their cleavage properties, and the conditions in which they may be used to cleave genomic DNA are very well known in the art and may be obtained from the catalogs of virtually any supplier of laboratory reagents (e.g., New England Biolabs, Berverly, MA or Stratagene, La Jolla, CA).

Accordingly, a subject genomic source may be cleaved by known methods to produce a sample containing nucleic acid fragments.

### Terminal labeling

In the subject method, nucleic acid fragments produced by one of the cleavage methods described above are end-labeled, i.e., terminally labeled, to provide a population of labeled nucleic acids having a labeled 3' or 5' terminus. In other words, the population of nucleic acids produced by these methods is labeled at their 5' ends or their 3' ends, but not at both ends. In general, the subject population of labeled nucleic acids are not "internally labeled", where "internally labeled" nucleic acids are nucleic acids that contain labeled nucleotides that are not at the 3' or 5' termini of the nucleic acids. The terminus of a nucleic acid encompasses the terminal nucleotide (i.e., the G, A, T or C that lies at the absolute end of the molecule), as well up to about 10-15 nucleotides (e.g., about 9, 5 or 3 nucleotides) that lie immediately adjacent thereto. In other words, a terminus of a subject nucleic acid is represented by up to about 10, up to about 6, up to about 4 or up to about 2 contiguous nucleotides or a single terminal nucleotide at the immediate end of a nucleic acid molecule. In certain embodiments, the terminus of a nucleic acid may be termed a "terminal region" of a nucleic acid.

In general, methods for end-labeling nucleic acid fragments, which may be double stranded or single stranded-labeling methods, according to the cleavage method used and whether or not the nucleic acids have been subjected to denaturing conditions, for example), are well known in the art (see, e.g., Ausubel et al, Short Protocols in Molecular Biology, 5th ed., Wiley & Sons, 2002 and Sambrook et al., Molecular Cloning: A Laboratory Manual, Third Edition, (2001) Cold Spring Harbor, N.Y.) and, as such, need not be described herein in any more detail than that set forth below.

In many embodiments, the subject labeling methods may employ a DNA polymerase, such as a template-dependent polymerase (e.g., T4 DNA polymerase, Taq polymerase, reverse transcriptase (MMLV RT), the Klenow fragment of DNA polymerase I and the like) or a template-independent DNA polymerase (such as terminal transferase), a polynucleotide ligase (such as T4 DNA ligase), or any combination thereof. As would be recognized by one of skill in the art, a wide variety of DNA polymerases and ligases employable in the subject methods are available.

In general, the subject labeling methods involve subjecting the nucleic acid fragments to conditions suitable for end-labeling, where such conditions are those that provide for labeling of the 3' or 5' end of the nucleic acid fragments, as desired. Once a particular enzyme for labeling is chosen, the particular labeling conditions that may be employed in the subject methods will be apparent. The subject labeling methods may be used to label a terminus of the subject nucleic acid fragments with a pre-determined number of up to about 5 or 10 or more label molecules or "dye" molecules (e.g., 1, 2, 3, 4 or 5 label molecules). In certain embodiments, each of the end-labeled nucleic acids contain the same number of label molecules.

Labels that find use in the subject invention include fluorescent labels, i.e., labels that contain a fluorophore moiety. Specific fluorescent labels of interest include: xanthene dyes, e.g. fluorescein and rhodamine dyes, fluorescein isothiocyanate (FITC), 6-carboxyfluorescein (commonly known by the abbreviations FAM and F),6-carboxy-2',4',7',4,7-hexachlorofluorescein (HEX), 6-carboxy-4', 5'-dichloro-2', 7'-dimethoxyfluorescein (JOE or J), N,N.N',N'-tetramethyl-6-carboxyrhodamine (TAMRA or T), 6-carboxy-X-rhodamine (ROX or R), 5-carboxyrhodamine-6G (R6G⁵ or G⁵), 6-carboxyrhodamine-6G (R6G⁶ or G⁶), and rhodamine 110; cyanine dyes, e.g. Cy3, Cy5 and Cy7 dyes; coumarins, e.g umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3, Cy5, etc; BODIPY dyes, ALEXA dyes and quinoline dyes. Specific fluorophores of interest that are commonly used in subject applications include: Pyrene, Coumarin, Diethylaminocoumarin, FAM, Fluorescein Chlorotriazinyl, Fluorescein, R110, Eosin, JOE, R6G, Tetramethylrhodamine, TAMRA, Lissamine, ROX, Napthofluorescein, Texas Red, Napthofluorescein, Cy3, and Cy5, etc. Further suitable fluorescent labels may be found in Kricka et al. (Ann Clin Biochem. 39:114-29, 2002). Such labels are generally covalently linked to a nucleotide that is either incorporated into a nucleic acid fragment using a polymerase, or ligated to a nucleic acid fragment using a ligase, as desired. Nucleotides labeled with fluorescent labels are well known in the art.

In order to exemplify this aspect of the invention, several labeling protocols suitable for employment in the subject methods are set forth below. These examples are non-limiting and should not be interpreted as limiting the invention in any way.

### 3'end labeling methods

In certain embodiments, it is the 3' termini of the nucleic acid fragments that are labeled. Labeling of the 3' terminus of a nucleic acid may be accomplished, for example, by:
1) Contacting a sample of nucleic acid fragments containing 5' overhangs (i.e., a population of nucleic acid fragments produced by cleaving a genomic source with a restriction enzyme that digests DNA to produce a 5' overhang), with a template dependent DNA polymerase, e.g., Klenow, reverse transcriptase, or T4 DNA polymerase, in the presence of labeled nucleotides. The polymerase extends the recessed 3' ends of the duplexed nucleic acids and incorporates at least one labeled nucleotide into the extension product to produce a nucleic acid labeled at its 3' end. Depending on the desired outcome, the exact conditions used (e.g., whether only labeled nucleotides or a mixture of unlabeled and labeled nucleotides are used in the polymerase reaction) and the sequence of the 5' overhang, each of the labeled nucleic acids may contain 1, 2, 3 or 4 label molecules which may be conjugated to the same or different nucleotide monomers. In another example, a labeled dideoxynucleotide may be incorporated using this method to provide labeled nucleic acids each containing exactly 1 label molecule.
2) Contacting a sample of single- or double-stranded nucleic acid fragments cleaved by any means (sometimes, but not always, containing a 3' overhang) with a terminal transferase in the presence of labeled nucleotides. The terminal transferase extends the 3' ends of the nucleic acid fragments and incorporates at least one, usually up to about 10, labeled nucleotides. Since a mixture of labeled and unlabeled nucleotides may be used during extension, the number of labeled nucleotides incorporated into a nucleic acid can be controlled.
3) Contacting a sample of single- or double-stranded nucleic acid fragments cleaved by any means (sometimes, but not always, containing a 3' overhang) with a terminal transferase in the presence of a labeled dideoxynucleotide (a ddNTP) to produce labeled nucleic acid fragments each containing a single label molecule.
4) Contacting a sample containing blunt-end double-stranded nucleic acid fragments (i.e., a population of nucleic acid fragments produced by digestion of a genomic source with a restriction enzyme that cleaves to produce blunt ends) with *Taq* DNA polymerase in the presence of labeled dATP or ddATP. As is well known in the art, under such conditions *Taq* DNA polymerase extends the 3' ends of the nucleic acids with a single dATP or ddATP molecule to produce nucleic acid fragments each containing a single label molecule.
5) Contacting a sample of double-stranded nucleic acid fragments cleaved by any means with a T4 DNA polymerase in the presence of labeled nucleotides. As is well known, T4 DNA polymerase possesses a 3' exonuclease activity as well a 5'-3' DNA polymerase activity. Accordingly, in a single reaction, the 3' exonuclease activity of T4 DNA polymerase "chews back", i.e., removes, up to about 10 nucleotide monomers from the 3' end of a nucleic acid fragment, and extends i.e., "fills in", the 3' end of the remaining fragment to produce a 3' end that is flush with the 5' end of the duplexed nucleic acid molecule. T4 DNA polymerase can incorporate labeled dNTPs into the 3' end of a nucleic acid during the "fill in" reaction to produce a nucleic acid fragment labeled at its 3' terminus. Again, a mixture of labeled and unlabeled nucleotides may be used in such a reaction to control the number of labeled nucleotides incorporated into to the nucleic acid fragments.
6) Contacting a sample of nucleic acid fragments containing a 5' overhang with a labeled oligonucleotide having a 5' end that is complementary to the sequence of the 5' overhang, in the presence of a ligase. The ligase joins the labeled oligonucleotide onto the 3' end of the nucleic acid fragments to produce a nucleic acid that is 3' end labeled. In particular embodiments, oligonucleotides containing a 5' phosphate group but lacking a 3' hydroxyl group may be used in this method to avoid ligation of the oligonucleotides to the 5' ends of the nucleic acid fragments, and self-ligation of the oligonucleotides. Since an oligonucleotide may be made to contain any number of label molecules, the number of label molecules present in each labeled nucleic acid may be controlled.

In summary, the nucleic acids may be labeled by: a) cleaving the genomic source using a 5' -overhang-producing restriction endonuclease and adding a labeled nucleotide to the 3' ends of the nucleic acid fragments using a template-dependent DNA polymerase; b) adding a labeled nucleotide to the 3' ends of the nucleic acid fragments using a terminal transferase; c) adding a labeled dideoxynucleotide to the 3' ends of the nucleic acid fragments using a terminal transferase; d) cleaving the genomic source using a blunt end-producing restriction endonuclease and adding a labeled nucleotide to the 3' ends of the nucleic acid fragments using Taq polymerase; e) adding a labeled nucleotide to the 3' ends of the nucleic acid fragments using T4 DNA polymerase; or f) adding a 3' end-labeled oligonucleotide onto the 3' ends of the nucleic acids using a ligase.

### 5'end labeling methods

In certain embodiments, it is the 5' terminus of the nucleic acid fragments that is labeled. Labeling of the 5' terminus of a nucleic acid may be accomplished, for example, by:
1) Contacting a sample of nucleic acid fragments containing a 3' overhang with a labeled oligonucleotide having a 3' end that is complementary to the sequence of the 3' overhang, in the presence of a ligase. The ligase joins the labeled oligonucleotide onto the 5' end of the nucleic acid fragments to produce a nucleic acid that is 5' end labeled. In particular embodiments, oligonucleotides containing a 3' hydroxyl group but lacking a 5' phosphate group may be used in this method to avoid ligation of the oligonucleotides to the 3' ends of the nucleic acid fragments, and self-ligation of the oligonucleotides. Since a labeled oligonucleotide may be produced containing a defined number of label molecules present thereon, the number of label molecules present in each labeled nucleic acid may be controlled.
2) Contacting a sample of double or single stranded nucleic acid fragments made by any method with a labeled primer under primer extension conditions. The primer may be a random primer, or a mixture of primers with specific sequences. Again, since a labeled primer may be produced containing a defined number of label molecules present thereon, the number of label molecules present in each labeled nucleic acid may be controlled.

Several other end-labeled methods that could be used in the subject methods would immediately become apparent to one of skill in the art in view of the disclosure set forth above.

In certain embodiments, a pair of genomic sources, typically a "test" and "reference" pair of genomic sources, will be labeled with "distinguishable" labels in that the labels can be independently detected and measured, even when the labels are mixed. In other words, the amounts of label present (e.g., the amount of fluorescence) for each of the labels are separately determinable, even when the labels are co-located (e.g., in the same tube or in the same duplex molecule or in the same feature of an array). Suitable distinguishable fluorescent label pairs useful in the subject methods include Cy-3 and Cy-5 (Amersham Inc., Piscataway, NJ), Quasar 570 and Quasar 670 (Biosearch Technology, Novato CA), Alexafiluor555 and Alexafluor647 (Molecular Probes, Eugene, OR), BODIPY V-1002 and BODIPY V 1005 (Molecular Probes, Eugene, OR), POPO-3 and TOTO-3 (Molecular Probes, Eugene, OR), fluorescein and Texas red (Dupont, Bostan MA) and POPRO3 TOPRO3 (Molecular Probes, Eugene, OR). Further suitable distinguishable detectable labels may be found in Kricka et al. (Ann Clin Biochem. 39:114-29, 2002).

In labeling a pair of genomic sources with distinguishable labels, the pair of genomic sources is usually cleaved and labeled with identical methods, except for the use of different labels in the methods.

Accordingly, the subject labeling methods may be used to provide a population of nucleic acids having a terminal label at an end that is distal from a substrate surface when the nucleic acids are hybridized to nucleic acids immobilized thereto.

### ARRAY-BASED CGH ASSAY METHODS

As discussed above, the population of labeled nucleic acids produced by the above methods find particular use in array-based CGH assays. A description of exemplary CGH assays in which the subject methods find use is set forth below.

### Array platforms

Array platforms for performing the subject methods are generally well known in the art (e.g., see Pinkel et al., Nat. Genet. (1998) 20:207-211; Hodgson et al., Nat. Genet. (2001) 29:459-464; Wilhelm et al., Cancer Res. (2002) 62: 957-960) and, as such, need not be described herein in any great detail.

In general, arrays suitable for use in performing the subject methods contain a plurality (i.e., at least about 100, at least about 500, at least about 1000, at least about 2000, at least about 5000, at least about 10,000, at least about 20,000, usually up to about 100,000 or more) of addressable features containing polynucleotides that are linked to a usually planar solid support via one terminus of the polynucleotide molecules (i.e. either the 3' or 5' end). Features on a subject array usually contain a polynucleotide that hybridizes with, i.e., specifically binds to, genomic sequences from a cell. Accordingly, such "comparative genome hybridization arrays", for short "CGH arrays" typically have a plurality of different BACs, cDNAs, oligonucleotides, or inserts from phage or plasmids, etc., that are addressably arrayed. In many embodiments, the subject CGH arrays contain single-stranded polynucleotides bound to the surface of a substrate at one end of the polynucleotides. As such, CGH arrays usually contain surface bound polynucleotides that are about 10-25 bases in length, 15-80 bases in length, about 20-100 bases in length, about 100-1000 bases in length, or about 200-5000 bases in length, depending on the platform used. Suitable array platforms for CGH experiments, and exemplary methods by which such assays may be performed, are described in issued U.S. patents 6,465,182; 6,335,167; 6,251,601; 6,210,878; 6,197,501; 6,159,685; 5,965,362; 5,830,645; 5,665,549; 5,447,841 and 5,348,855, which patents are incorporated by reference in their entirety.

In particular embodiments, CGH arrays containing surface-bound oligonucleotides, i.e., oligonucleotides of about 20 to about 100 nucleotides and up to about 200 nucleotides in length, e.g., 40-65 nucleotides in length, find particular use in the subject methods.

### Methods

The methods described above are generally useful in methods of assessing copy number of a genomic region, where a genomic region is generally of any length equal to or greater than the length of a surface bound polynucleotide corresponding to that region. In certain embodiments, genomic regions are greater than about 20 bp, between about 500 bp and the length of an intact chromosome, e.g., any about 1 kb to about 1 Mbp or about 10 kb to about 1000 kb region of a genome. In general, the methods involve contacting a first population of end-labeled nucleic acids made from a genomic source for a test cell with an array of surface-immobilized polynucleotides under conditions that provide for specific hybridization, and evaluating binding of the end-labeled nucleic acids for binding to the surface-immobilized polynucleotides. In certain embodiments, evaluating is done relative to binding of a reference population of end-labeled nucleic acids made from a genomic source for a reference cell.

In general, the subject array CGH assays involve labeling a test and reference genomic source to make two labeled populations of nucleic acids which may be distinguishably labeled, contacting the labeled populations of nucleic acids with at least one array of surface bound polynucleotides under specific hybridization conditions, and analyzing any data obtained from hybridization of the nucleic acids to the surface bound polynucleotides. Such methods are generally well known in the art (see, e.g., Pinkel et al. (Nat. Genet. (1998) 20:207-211); Hodgson et al. (Nat. Genet. (2001) 29:459-464); Wilhelm et al. (Cancer Res. (2002) 62: 957-960)) and, as such, need not be described herein in any great detail.

The above-described labeling reactions produce a first and second population of end-labeled nucleic acids that correspond to the test and reference cells, respectively. After nucleic acid purification and any pre-hybridization steps to suppress repetitive sequences (e.g., hybridization with Cot-1 DNA), the populations of end-labeled nucleic acids are contacted to an array of surface bound polynucleotides, as discussed above, under conditions such that nucleic acid hybridization to the surface bound polynucleotides can occur, e.g., in a buffer containing 50% formamide, 5×SSC and 1% SDS at 42°C, or in a buffer containing 5xSSC and 1% SDS at 65°C, both with a wash of 0.2xSSC and 0.1% SDS at 65°C.

The labeled nucleic acids can be contacted to the surface bound polynucleotides serially, or, in other embodiments, simultaneously (i.e., the labeled nucleic acids are mixed prior to their contacting with the surface-bound polynucleotides). Depending on how the nucleic acid populations are labeled (e.g., if they are distinguishably or indistinguishably labeled), the populations may be contacted with the same array or different arrays. Where the populations are contacted with different arrays, the different arrays are substantially, if not completely, identical to each other in terms of target feature content and organization.

Standard hybridization techniques (using high stringency hybridization conditions) are used to hybridize a labeled sample to a target nucleic acid array. Suitable methods are described in references describing CGH techniques (Kallioniemi et al., (Science 258:818-821 (1992)) and WO 93/18186). Several guides to general techniques are available, e.g., Tijssen, Hybridization with Nucleic Acid Probes, Parts I and II (Elsevier, Amsterdam 1993). For a descriptions of techniques suitable for in situ hybridizations see, Gall et al. (Meth. Enzymol., 21:470-480 (1981)) and Angerer et al. (in Genetic Engineering: Principles and Methods Setlow and Hollaender, Eds. Vol 7, pgs 43-65 (plenum Press, New York 1985)). See also United States Patent Nos: 6,335,167; 6,197,501; 5,830,645; and 5,665,549; the disclosures of which are herein incorporate by reference.

Generally, comparative genome hybridization methods comprise the following major steps: (1) immobilization of polynucleotides on a solid support by one end of the polynucleotide; (2) pre-hybridization treatment to increase accessibility of support-bound polynucleotides and to reduce nonspecific binding; (3) hybridization of a mixture of distal end labelednucleic acids to the surface-bound nucleic acids, typically under high stringency conditions; (4) post-hybridization washes to remove nucleic acid fragments not tightly bound to the solid support polynucleotides; and (5) detection of the hybridized labeled nucleic acids. The reagents used in each of these steps and their conditions for use vary depending on the particular application.

As indicated above, hybridization is carried out under suitable hybridization conditions, which may vary in stringency as desired. In certain embodiments, highly stringent hybridization conditions may be employed. The term "high stringency hybridization conditions" as used herein refers to conditions that limit nucleic acid binding complexes on an array surface to specifically complementary binding members, i.e., between the surface-bound polynucleotides and complementary labeled nucleic acids in a sample. Representative high stringency assay conditions that may be employed in these embodiments are provided above.

The above hybridization step may include mixing of the immobilized polynucleotides and the sample of labeled nucleic acids, where the mixing may be accomplished using any convenient protocol, e.g., shaking, rotating, spinning, and the like.

Following hybridization, the array-surface bound polynucleotides are typically washed to remove unbound and not tightly bound labeled nucleic acids. Washing may be performed using any convenient washing protocol, where the washing conditions are typically stringent, as described above.

Following hybridization and washing, as described above, the hybridization of the labeled nucleic acids to the targets is then detected using standard techniques so that the surface of immobilized targets, e.g., the array, is read. Reading of the resultant hybridized array may be accomplished by illuminating the array and reading the location and intensity of resulting fluorescence at each feature of the array to detect any binding complexes on the surface of the array. For example, a scanner may be used for this purpose, which is similar to the AGILENT MICROARRAY SCANNER available from Agilent Technologies, Palo Alto, CA. Other suitable devices and methods are described in U.S. patent applications: Serial No. 09/846125 "Reading Multi-Featured Arrays" by Dorsel et al. (published as US2002/0160369 and US 6,756,202); and United States Patent No. 6,406,849, which references are incorporated herein by reference. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). In the case of indirect labeling, subsequent treatment of the array with the appropriate reagents may be employed to enable reading of the array. Some methods of detection, such as surface plasmon resonance, do not require any labeling of nucleic acids, and are suitable for some embodiments.

Results from the reading or evaluating may be raw results (such as fluorescence intensity readings for each feature in one or more color channels) or may be processed results (such as those obtained by subtracting a background measurement, or by rejecting a reading for a feature which is below a predetermined threshold, normalizing the results, and/or forming conclusions based on the pattern read from the array (such as whether or not a particular target sequence may have been present in the sample, or whether or not a pattern indicates a particular condition of an organism from which the sample came).

In certain embodiments, the subject methods include a step of transmitting data or results from at least one of the detecting and deriving steps, also referred to herein as evaluating, as described above, to a remote location. By "remote location" it is meant a location other than the location at which the array is present and the hybridization occurred. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

Accordingly, a pair of genomic sources may be labeled to make two populations of end-labeled nucleic acids, the nucleic acids contacted with an array of surface-bound polynucleotides, and the level of labeled nucleic acids bound to each surface-bound polynucleotide assessed.

In certain embodiments, a surface-bound polynucleotide is assessed by determining the level of binding of the population of labeled nucleic acids to that polynucleotide. The term "level of binding" means any assessment of binding (e.g. a quantitative or qualitative, relative or absolute assessment) usually done, as is known in the art, by detecting signal (i.e., pixel brightness) from the label associated with the labeled nucleic acids. Since the level of binding of labeled nucleic acid to a surface-bound polynucleotide is proportional to the surface density of bound label, the level of binding of labeled nucleic acid is usually determined by assessing the amount of label associated with the surface-bound polynucleotide.

In certain embodiments, a surface-bound polynucleotide may be assessed by evaluating it's binding to two populations of nucleic acids that are distinguishably labeled. In these embodiments, for a single surface-bound polynucleotide of interest, the results obtained from hybridization with a first population of labeled nucleic acids may be compared to results obtained from hybridization with the second population of nucleic acids, usually after normalization of the data. The results may be expressed using any convenient means, e.g., as a number or numerical ratio, etc.

By "normalization" is meant that data corresponding to the two populations of nucleic acids are globally normalized to each other, and/or normalized to data obtained from controls (e.g., internal controls produce data that are predicted to equal in value in all of the data groups). Normalization generally involves multiplying each numerical value for one data group by a value that allows the direct comparison of those amounts to amounts in a second data group. Several normalization strategies have been described (Quackenbush et al (Nat Genet. 32 Suppl:496-501, 2002), Bilban et al (Curr Issues Mol Biol. 4:57-64, 2002), Finkelstein et al (Plant Mol Biol.48(1-2):119-31, 2002), and Hegde et al (Biotechniques. 29:548-554, 2000)). Specific examples of normalization suitable for use in the subject methods include linear normalization methods, non-linear normalization methods, e.g., using lowess local regression to paired data as a function of signal intensity, signal-dependent non-linear normalization, qspline normalization and spatial normalization, as described in Workman et al. (Genome Biol. 2002 3, 1-16). In certain embodiments, the numerical value associated with a feature signal is converted into a log number, either before or after normalization occurs. Data may be normalized to data obtained using the data obtained from a support-bound polynucleotide for a chromosome of known concentration in any of the chromosome compositions.

Accordingly, binding of a surface-bound polynucleotide to an end-labeled population of nucleic acids may be assessed. In most embodiments, the assessment provides a numerical assessment of binding, and that numeral may correspond to an absolute level of binding, a relative level of binding, or a qualitative (e.g., presence or absence) or a quantitative level of binding. Accordingly, a binding assessment may be expressed as a ratio, whole number, or any fraction thereof.

In other words, any binding may be expressed as the level of binding of a surface-bound polynucleotide to an end-labeled population of nucleic acids made from a test genomic source, divided by its level of binding to an end-labeled population of nucleic acids made from a reference genomic source (or vice versa).

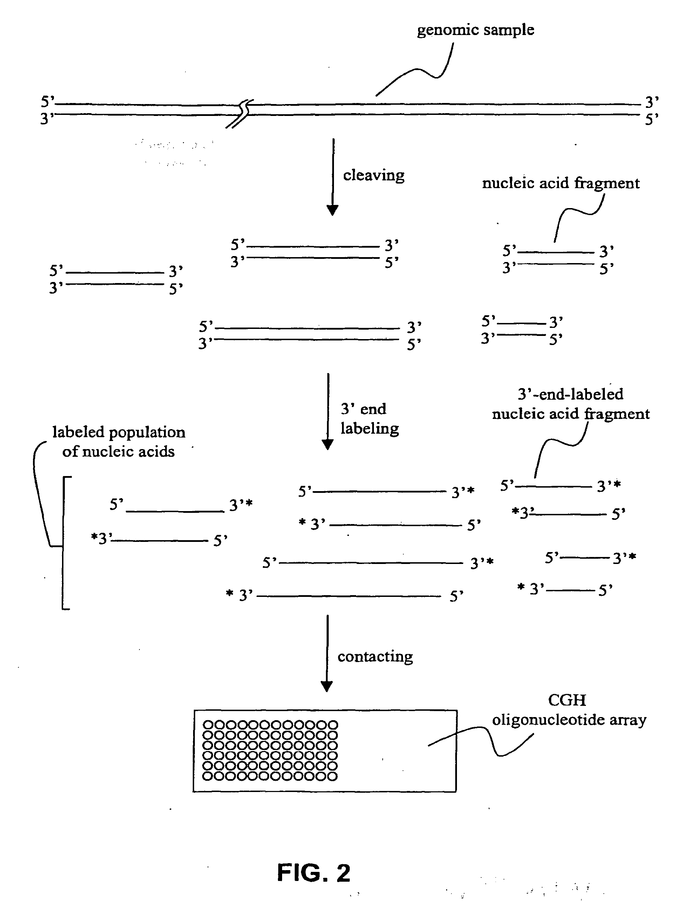

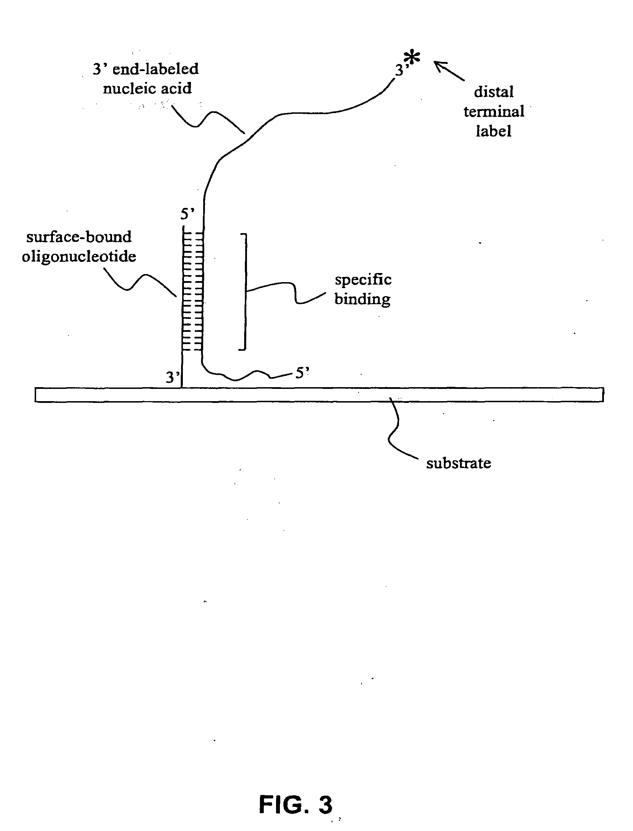

In an exemplary embodiment shown in Fig. 2, a genomic sample is cleaved into nucleic acid fragments and the nucleic acid fragments are 3' end-labeled to produce a population of nucleic acids labeled at their 3' terminus. The population of end-labeled nucleic acids is contacted with an oligonucleotide CGH array in which the oligonucleotides are anchored to the surface of a substrate by their 3' ends, under stringent binding conditions. The array is read to produce data. An exemplary binding complex produced according to the methods of the invention is shown in Fig. 3. In this figure, a 3' end-labeled nucleic acid is specifically bound to a surface-bound oligonucleotide that is tethered to the surface of the substrate by its 3' end. The label, indicted by an asterisk ("*"), is present at the distal terminus of the end-labeled nucleic acid, and, as such, more distant to energy receptor molecules than labels present on labeled nucleic acids made by other methods, and, as such, are subject to less quenching than labels present on labeled nucleic acids made by other methods.

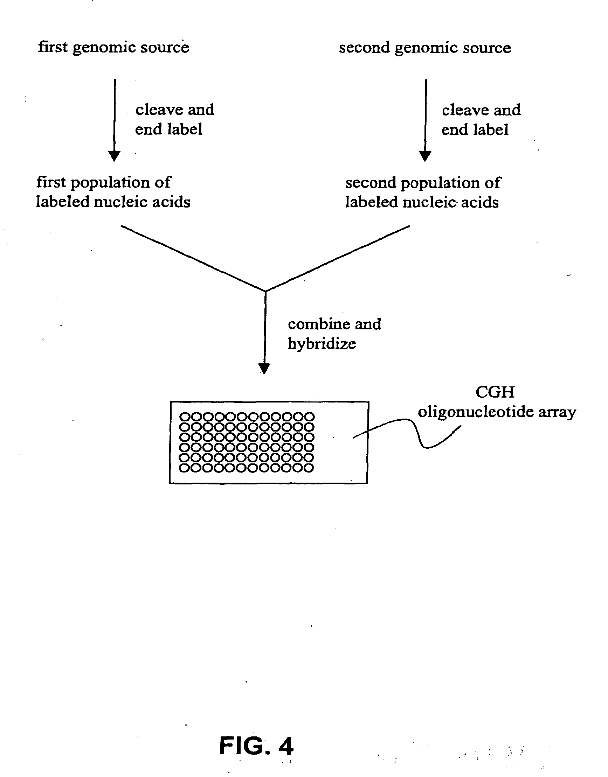

Fig. 4 shows an exemplary CGH assay using the methods described above. A first and second genomic sources are cleaved and distinguishably labeled using the method set forth above to produce a first and second population of labeled nucleic acids, and the populations of labeled nucleic acids are combined and contacted with an oligonucleotide CGH array under conditions that provide for stringent hybridization. The array is then read to produce data.

Accordingly, the invention also provides a system for performing an array-based CGH assay. In many embodiments, the system contains reagents for cleaving a genomic source into nucleic acid fragments, reagents for end-labeling a terminus of those fragments, and a CGH array. The components of the subject system depend on the exact method used, and, as such, the components may vary.

Further, the invention provides an array of surface-bound polynucleotides (e.g. oligonucleotides) and a population of end-labeled nucleic acids having a terminal label. The end-labeled nucleic acids are derived from a genomic source and are complexed with said surface-bound polynucleotides such that the terminal label is distal to the surface.

### KITS

Also provided by the subject invention are kits for practicing the subject methods, as described above. The subject kits at least include reagents for cleaving a genomic source into nucleic acid fragments, reagents for end-labeling a terminus of those fragments, and hybridization buffer suitable for use with a CGH array. Other optional components of the kit include: a CGH array. In some embodiments, arrays may be included in the kits. The various components of the kit may be present in separate containers or certain compatible components may be precombined into a single container, as desired.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

In addition to the subject database, programming and instructions, the kits may also include one or more control analyte mixtures, e.g., two or more control analytes for use in testing the kit. **UTILITY**

The subject methods find most application in CGH assays, e.g., any application in which one wishes to compare the copy number of nucleic acid sequences found in at least one genomic sample, e.g., two or more genomic samples. Protocols involving self-comparison of genomic samples are encompassed by the subject methods.

One type of representative application in which the subject CGH arrays find use is the quantitative comparison of copy number of one nucleic acid sequence in a first collection of nucleic acid molecules relative to the copy number of the same sequence in a second collection.

As such, the present invention may be used in methods of comparing abnormal nucleic acid copy number and mapping of chromosomal abnormalities associated with disease. In many embodiments, the subject methods are employed in applications that use polynucleotides immobilized on a solid support, to which differentially labeled nucleic acids produced as described above are hybridized. Analysis of processed results of the described hybridization experiments provides information about the relative copy number of nucleic acid domains, e.g. genes or regions thereof, in genomes.

Such applications compare the copy numbers of sequences capable of binding to the target elements. Variations in copy number detectable by the methods of the invention may arise in different ways. For example, copy number may be altered as a result of amplification or deletion of a chromosomal region, e.g. as commonly occurs in cancer.

Representative applications in which the subject methods find use are further described in U.S. Patent Nos. 6,335,167; 6,197,501; 5,830,645; and 5,665,549; the disclosures of which are herein incorporated by reference.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The disclosures in United States patent application No. 10/964,505, from which this application claims priority, and in the abstract accompanying this application are incorporated herein by reference.

## Claims

1. A method of producing a population of labeled nucleic acids for use in a comparative genome hybridization (CGH) assay that employs a substrate having surface immobilized polynucleotides, comprising:
cleaving a genomic source to produce a sample containing nucleic acid fragments; and
end-labeling said nucleic acid fragments to provide a population of nucleic acids having a terminal label at an end that is distal to said substrate surface when said nucleic acids are hybridized to said surface immobilized polynucleotides.

2. A method as claimed in claim 1, wherein said nucleic acid fragments are end-labeled at their 3' terminus and said surface immobilized polynucleotides are bound to said substrate by their 3' terminus.

3. A method as claimed in claim 1, wherein said nucleic acid fragments are end-labeled at their 5' terminus and said surface immobilized polynucleotides are bound to said substrate by their 5' terminus.

4. A method as claimed in claim 1, 2 or 3, wherein said cleaving employs a restriction enzyme.

5. A method as claimed in any preceding claim, wherein said end-labeling employs a polymerase, a reverse transcriptase, or a ligase.

6. A method as claimed in any preceding claim, wherein said method employs cleaving said genomic source using a restriction enzyme and adding a labeled nucleotide to an end of said nucleic acid fragments using a polymerase.

7. A comparative genome hybridization (CGH) assay, comprising:
preparing an end-labeled population of nucleic acids according to the method of any of claims 1 to 6;
contacting said end-labeled labeled population of nucleic acids with an array comprising substrate surface-bound polynucleotides to provide polynucleotide/nucleic acid hybridization complexes in which said terminal label of said end-labeled nucleic acids is distal from said substrate surface; and
assessing binding of said end-labeled population of nucleic acids to said array.

8. A kit comprising:
reagents for cleaving a genomic source into nucleic acid fragments;
reagents for end-labeling a terminus of said fragments; and
hybridization buffer suitable for use in an array-based CGH assay.

9. A system for performing a CGH assay, comprising:
reagents for cleaving a genomic source into nucleic acid fragments;
reagents for end-labeling a terminus of said fragments; and
a CGH array.

10. A composition comprising:
an array of surface-bound polynucleotides; and
a population of end-labeled nucleic acids having a terminal label;
wherein said end-labeled nucleic acids are derived from a genomic source and are complexed with said surface-bound polynucleotides such that said terminal label is distal from said surface.
